## Europäisches Patentamt
## European Patent Office
## Office européen des brevets

(19)

(11) Publication number: **0 050 525**
**B1**

(12) **EUROPEAN PATENT SPECIFICATION**

(45) Date of publication of patent specification: **06.06.84**

(21) Application number: **81304936.8**

(22) Date of filing: **21.10.81**

(51) Int. Cl.³: **C 01 B 33/28,**
**B 01 J 29/28, C 01 B 3/26**

(54) Synthetic modified crystalline silica.

(30) Priority: **22.10.80 GB 8034098**

(43) Date of publication of application:
**28.04.82 Bulletin 82/17**

(45) Publication of the grant of the patent:
**06.06.84 Bulletin 84/23**

(84) Designated Contracting States:
**BE DE FR GB IT NL**

(56) References cited:
**DE - A - 2 946 164**
**GB - A - 2 023 562**
**GB - A - 2 024 790**
**US - A - 3 654 185**
**US - A - 3 769 386**
**US - A - 3 770 845**
**US - A - 3 941 871**
**US - A - 4 046 859**
**US - E - 29 948**

(73) Proprietor: **The British Petroleum Company p.l.c.**
**Britannic House Moor Lane**
**London EC2Y 9BU (GB)**

(72) Inventor: **Ball, William John**
**The British Petroleum Company Ltd. Chertsey**
**Road**
**Sunbury-on-Thames Middlesex, TW16 7LN (GB)**
Inventor: **Stewart, David Gordon**
**The British Petroleum Company Ltd. Chertsey**
**Road**
**Sunbury-on-Thames Middlesex, TW16 7LN (GB)**

(74) Representative: **Harry, John et al,**
**c/o The British Petroleum Company plc Patents**
**Division Chertsey Road**
**Sunbury-on-Thames Middlesex TW16 7LN (GB)**

Courier Press, Leamington Spa, England.

# 0 050 525

**Description**

The present invention relates to a synthetic modified crystalline silica, a process for its production and its use as a catalyst.

In US—E—Re. 29948 there is described a crystalline metal organosilicate having a composition, in its anhydrous state, in terms of mole ratios of oxides as follows:—

$$0.9 \pm 0.2 \ [xR_2O+(1-x)M_{2/n}O]: 0.005 \ Al_2O_3: 1 \ SiO_2$$

wherein M is a metal other than a metal of Group IIIA, n is the valence of said metal, R is an alkylammonium radical and x is greater than 0 but not exceeding 1. The crystalline metal organosilicate may be synthesised by preparing a solution containing $(R_4N)_2O$, sodium oxide, an oxide of a metal other than a metal of Group IIIA and water in defined proportions and thereafter crystallising the mixture. The crystalline metal organosilicates as synthesised can have the original components thereof replaced by a wide variety of others according to techniques well known in the art. Typical replacing components would include hydrogen, ammonium, alkyl ammonium and aryl ammonium and metals other than metals of Group IIIA, including mixtures of the same. The hydrogen form may be prepared, for example, by substitution of original sodium with ammonium followed by calcination. With regard to the structure of the materials it is stated that regardless of the synthesised form of the organosilicate the spatial arrangement of atoms which form the basic crystal lattices remain essentially unchanged by the described replacement of sodium or other alkali metal or by the presence in the initial reaction mixture of metals in addition to sodium, as determined by an X-ray powder diffraction pattern of the resulting organosilicate. The aforesaid statements imply that the sodium or sodium in combination with other metal does not form an integral part of the structure of the crystalline organosilicate otherwise it would not be possible to exchange them with other components. Moreover the alkylammonium radical is an essential component of the crystalline material as synthesised and this also can be exchanged with other components.

GB—A—2024790 describes a silica-based material comprising crystalline silica which has been modified with one or more elements which have entered the crystalline lattice of the silica in place of the silica or in the form of salts of bisilicic or polysilicic acids. All those elements which form metallic cations are said to be useable for obtaining the modified silicas but preference is given to elements having, at least partially, an amphoteric character such as chromium, beryllium, titanium, vanadium, manganese, iron, cobalt, zinc, zirconium, rhodium, silver, tin, antimony and boron. The modified silicas of this kind are reported to be characterised by the presence of a single crystalline phase and to have the formula:—

$$(0.0001 \text{ to } 1) \ M_nO_m . SiO_2$$

wherein $M_nO_m$ is an oxide of the modifying element. The materials usually have a well defined crystalline structure as demonstrated by the X-ray diffraction spectra appearing in the specification. The silica-based synthetic materials are said to be useful as catalysts for a large number of reactions, including the conversion of dimethyl ether and/or methanol or other lower alcohols into hydrocarbons such as olefins and aromatic hydrocarbons.

GB—A—2023562 describes a crystalline silica or germania modified with aluminium or gallium, a process for its preparation and its use as a catalyst in a variety of reactions including the conversion of dimethyl ether and/or methanol or other lower alcohols to hydrocarbons (olefins and aromatics).

Furthermore EP—A—10572 discloses iron silicate having a zeolitic structure, a process for its production by crystallisation from a mixture of water glass and an aqueous solution of an amine at 100—200°C and in the presence of Fe(III) oxide and/or Fe(III) hydroxide and its use as a catalyst in cracking, hydrocracking and isomerisation processes.

We have previously found that contrary to the teaching of GB—A—2024790 synthetic cobalt-modified crystalline silica decomposes methanol to synthesis gas. This finding forms the subject of our EP—A—38682.

We have now synthesised a nickel-modified crystalline silica and have found that this also catalyses, inter alia, the decomposition of methanol to synthesis gas.

Accordingly, the present invention provides a synthetic modified crystalline silica comprising crystalline silica in which a proportion of the silicon atoms in the crystal lattice have been replaced by nickel, which modified crystalline silica has an X-ray powder diffraction pattern characterised by the lines substantially as set forth in Table 1.

The synthetic nickel-modified crystalline silica of the present invention has a characteristic X-ray powder diffraction pattern as shown in Table 1. The values in Table 1 were determined using standard techniques. The radiation was the K-alpha doublet of copper and a scintillation counter spectrometer with a strip chart pen recorder was used. The peak heights, I, and the positions as a function of 2 theta, where theta is the Bragg angle, were read from the spectrometer chart. From these the relative intensities 100×I/Io, where Io is the intensity of the strongest line or peak, and d, the interplanar

2

spacing in A corresponding to the recorded lines or peaks, were calculated. In Table 1, the relative intensities of the peaks are given in terms of symbols S=strong, M=medium, W=weak and VW=very weak.

TABLE 1

| D (Angstrom) | I/Io |
|---|---|
| 11.0 ±0.2 | S |
| 10.0 ±0.2 | S |
| 9.8 ±0.2 | S |
| 9.2 ±0.2 | W |
| 6.3 ±0.1 | VW |
| 5.9 ±0.1 | W |
| 5.85±0.1 | VW |
| 5.64±0.1 | VW |
| 5.50±0.1 | VW |
| 5.00±0.08 | VW |
| 4.90±0.08 | VW |
| 4.22±0.08 | VW |
| 3.83±0.07 | M |
| 3.80±0.07 | M |
| 3.79±0.07 | M |
| 3.73±0.05 | W |
| 3.69±0.05 | W |
| 3.64±0.05 | VW |
| 3.60±0.05 | VW |
| 3.42±0.05 | VW |
| 3.33±0.04 | VW |
| 3.30±0.04 | VW |
| 2.98±0.02 | VW |
| 2.95±0.02 | VW |

In another aspect the present invention also provides a process for the production of a synthetic modified crystalline silica which process comprises mixing, in a liquid medium comprising either water, an alcohol, or a mixture thereof, a source of silica, a source of nickel and an organic base in the absence of an alkali or alkaline earth metal compound and maintaining the mixture at elevated temperature for a time sufficient to effect crystallisation of the modified crystalline silica and recovering the modified crystalline silica so-formed.

Suitable sources of silica include, for example, sodium silicate, silica hydrosol, silica gel, silica sol and silicic acid. The preferred source of silica is an aqueous colloidal dispersion of silica particles. A suitable commercially available source of silica is Ludox Colloidal Silica marketed by Du Pont (Ludox is a Registered Trade Mark).

Suitable sources of nickel include the oxide, hydroxide, salts such as the nitrate and alkoxy derivatives such as the acetate.

Suitable organic bases include nitrogenous bases such as amines and substituted amines, e.g. alkanolamines. Preferably the nitrogenous base is a quaternary ammonium compound, which may be a tetraalkylammonium compound in which the alkyl group contains from 1 to 5 carbon atoms or a tetraarylammonium compound in which the aryl group is phenyl or an alkylphenyl group. A particularly suitable tetraalkylammonium compound is tetrapropylammonium hydroxide.

The amount of organic base employed may suitably be lower than the stoichiometric amount relative to silica and is preferably from 0.05 to 0.50 mole % per mole of silica.

The amounts of the source of silica and the source of nickel in the mixture may suitably be such that they provide a silicon to nickel atomic ratio greater than 5:1, preferably greater than 10:1.

The mixture may suitably be maintained at a temperature in the range from 100 to 220, preferably from 150 to 200°C for a period of from a few hours to several days, preferably for about 7 days. The mixture is preferably maintained at the requisite temperature in a closed vessel such as an autoclave under autogenous pressure.

The modified crystalline silica may suitably be recovered from the liquid medium, after cooling, by filtration. After recovery it is preferred to wash the crystalline material, suitably with boiling distilled water having dissolved therein an ammonium salt such as the nitrate or acetate.

Finally it is preferred to calcine the modified crystalline silica. Calcination may suitably be effected by heating in air at a temperature in the range from 300 to 700°C, preferably at about 550°C, for a period of from 2 to 24 hours.

The nickel-modified crystalline silica so produced may be used without any further modification as a catalyst in a variety of chemical reactions or there can be added other metals which are capable of imparting specific catalytic properties. Examples of such metals are platinum, palladium, nickel, cobalt, tungsten, rhodium, copper and zinc. The addition may suitably be effected by impregnation, using solutions of salts of the metals, e.g. nitrates, acetates, oxides or halides.

As mentioned hereinbefore the nickel-modified crystalline silica catalyses the decomposition of methanol to synthesis gas.

In another aspect therefore the invention provides a process for the production of synthesis gas from methanol which comprises contacting at elevated temperature methanol in the vapour phase with a catalyst comprising a nickel-modified crystalline silica as described hereinbefore.

Methanol is available commercially on a very large scale. It may be anhydrous or it may contain water up to, for example, 90% w/w. Alternatively water may be introduced as a separate feed in the form of steam. Furthermore the methanol may be diluted with carbon monoxide or carbon dioxide. Since the decomposition of methanol is endothermic the use of diluents offers a convenient method for introducing heat into the reaction.

The catalytic activity of the modified crystalline silica catalyst for methanol decomposition may be further enhanced by the addition of a metal of Group VIII of the Periodic Table either alone or in combination with one or more other metals of Groups I to VIII of the Periodic Table. The Periodic Table referred to is that contained in the Handbook of Chemistry and Physics, 44th Edition, published by the Chemical Rubber Publishing Company. Group VIII metals which are particularly effective include, for example, rhodium and cobalt, both alone and in combination with other metals. The Group VIII metal may be admixed with one or more other Group VIII metals and/or with metals of Groups I to VII of the Periodic Table, such as iron, copper, chromium, gold and zinc. A particularly preferred combination is a mixture of rhodium and copper. The metal or metals may be added by any of the known-techniques. Preferably the metal or metals are added by impregnating the modified crystalline silica with a solution of a compound of the metal or metals in a suitable solvent, such as water, and thereafter removing the solvent. The catalyst may suitably contain from 0.1 to 20%, preferably from 0.2 to 10%, by weight of the metal or metals. It will be appreciated that although the metal may be added in the form of a compound of the metal, under the conditions pertaining during the methanol decomposition process such compounds may well be chemically reduced.

Alternatively or in addition the modified crystalline silica may be mixed with other materials such as the amorphous aluminosilicate described in our copending European Application No. 79302108.0 (EP—A—9976) or dispersed on a supporting body such as clays, silica, alumina and silica/alumina.

The methanol may suitably be contacted with the modified crystalline silica catalyst at a temperature in the range from 200 to 600, preferably from 300 to 450°C, and at a pressure up to 100, preferably from 1 to 50 atmospheres. Since the decomposition of methanol is equilibrium limited it is preferred to employ high temperatures in combination with high pressures.

The process may be operated batchwise or continuously, continuous operation being preferred. The contact time, as hereinafter defined, for continuous operation may be up to 30, preferably from 1 to 5 seconds. For the purpose of this specification the contact time is defined as follows:—

$$\frac{\text{Volume of catalyst in millilitres}}{\text{Total volume of gas (in millilitres/second at NTP)}}$$

The catalyst may be used either as a fixed bed or as a fluidised bed.

The decomposition of methanol produces 2 volumes of hydrogen for every volume of carbon monoxide. This ratio can be altered if so desired by treating the hydrogen/carbon monoxide mixture in a manner well-known to those skilled in the art i.e. by the so-called "shift reaction".

The mixtures of hydrogen and carbon monoxide produced by the process of the present invention may be used in a variety of chemical reactions, including carbonylation, hydrocarbonylation and chemical reduction.

Alternatively the nickel-modified crystalline silica may be used as a catalyst for the conversion of synthesis gas at elevated temperature and pressure to hydrocarbons and/or oxygenated hydrocarbons.

Typically elevated temperatures in the range from 150 to 450°C, preferably from 200 to 400°C and elevated pressures in the range from 1 to 700 bars, preferably from 10 to 300 bars, may suitably be employed. The contact time for continuous operation may be up to 30 seconds and is preferably in the range from 0.01 to 5 seconds.

The catalytic activity of the modified crystalline silica may be enhanced by the addition of one or more metals in amounts suitably in the range from 0.1 to 20%, preferably from 0.2 to 10% by weight. Suitable metals include cobalt, iron, manganese, nickel, ruthenium, rhodium, palladium, zinc, chromium and copper.

The invention will now be particularly described by reference to the following Examples. In the Examples reference will be made to the molar yield of a particular product, to the CO conversion and to the selectivity. These terms are defined as follows:

4

$$\text{The molar yield of a particular product} = \frac{\text{Moles of methanol converted to a particular product}}{\text{Moles of methanol fed}} \times 100$$

$$\text{CO Conversion} = \frac{\text{Moles of carbon monoxide consumed}}{\text{Moles of carbon monoxide fed}} \times 100$$

$$\text{Selectivity} = \frac{\text{Moles of carbon monoxide converted to particular product}}{\text{Moles of carbon monoxide consumed}} \times 100$$

Preparation of the modified crystalline silica

Example 1

Nickel nitrate hexahydrate (2.9 g) was dissolved in deionised water (22.8 g). Tetrapropyl ammonium hydroxide (22.9 g, 20% w/w) was mixed with Ludox silica (21.8 g containing 40% silica) and the above nickel nitrate solution was added with stirring. The gel was allowed to stand for one hour, transferred to a pressure vessel and heated at 170°C for 50 hours with rotation. The vessel was cooled to room temperature, the contents filtered and the filter cake washed three times by heating with 1 molar ammonium chloride solution (3×150 ml). Finally the solid was washed with deionised water (150 ml), dried at 120°C and calcined at 500°C for 16 hours. The material was shown to be crystalline and the X-ray diffraction data are given in Table 2. Analysis of the crystalline solid showed 41.4% wt/wt silicon and 5.3% wt/wt nickel.

Example 2

The procedure of Example 1 was repeated using nickel nitrate hexahydrate (0.9 g), tetrapropylammonium hydroxide (22.9 g), 20% wt/wt), Ludox silica (21.8 g, containing 40% silica) and deionised water (22.8 g). The material was shown to be X-ray crystalline and the X-ray diffraction data are given in Table 3. Analysis of the solid showed 41.9% wt/wt silicon and 1.39% wt/wt nickel.

Use of the modified crystalline silicas as catalysts

Example 3

The decomposition of methanol into carbon monoxide and hydrogen (synthesis gas)

A gaseous feed of methanol was passed over the modified crystalline silica product of Example 1 contained in a glass reactor at 400°C and 2.8 seconds contact time.

The molar yield of carbon monoxide based on the total methanol feed was 88%. The remaining products were methane and dimethyl ether.

The hydrogen to carbon monoxide ratio of the synthesis gas obtained was approximately 2:1.

Example 4

The conversion of synthesis gas into hydrocarbons and oxygenated hydrocarbons

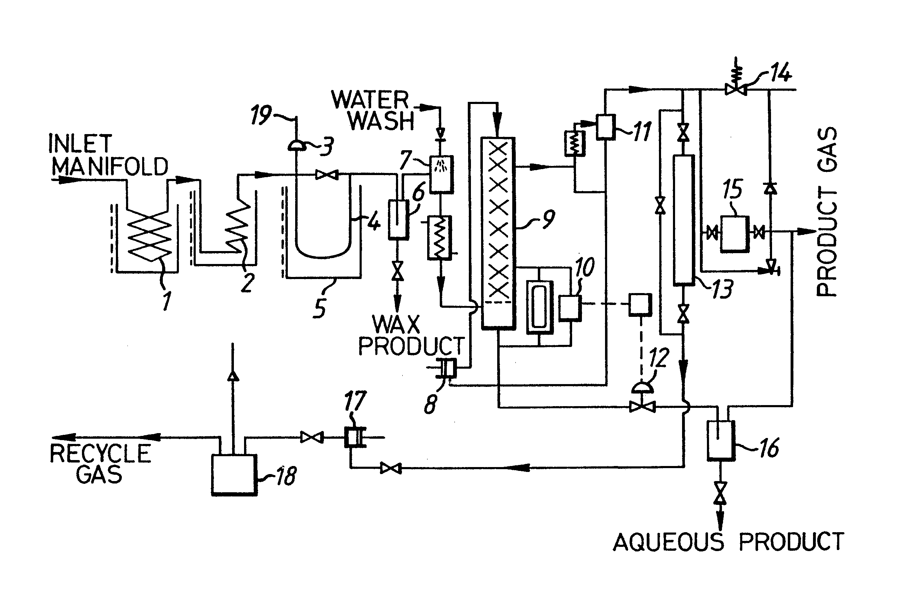

With reference to the accompanying Figure a mixture of carbon monoxide and hydrogen in a molar ratio of 1:2 was passed via the inlet manifold through the two preheater coils (1) and (2) maintained at 150°C and 200°C respectively in silicone oil baths. The heated gases were then fed via a heat-traced line to the copper-lined reactor (4) containing a fixed bed of the nickel silicate catalyst, prepared in the manner described above, in the form of 1/16 in. extrudate. The reactor was maintained at the desired reaction temperature by immersion in a molten salt bath (5). The product gases were passed via a heat-traced line through a knock-out pot for wax products (6) to a small quench vessel (7) into the top of which water was sprayed. The gases were then passed through a water cooler to the bottom of the water wash tower (9) which was packed with 3/8 inch Raschig rings. In the tower (9) the product gases were washed counter-current with water. The resulting liquid product was fed into the receiver (16) and any dissolved gases were recombined with the product gas stream from the back pressure regulator (15). The separated gas stream from the top of the water wash tower (9) was passed through a water cooler to the knock-out pot (11) and then to the inlet side of the dome-loaded back pressure regulator (15). Recycle gas was recovered from the inlet side of the back pressure regulator (15), passed through a molecular sieve drier (13) and compressed up to 67 bars in the gas ballast vessel (18) using the gas recycle pump (17). The recycle gas was fed back to the inlet manifold. Provision was made to feed spot samples of the inlet gases and the total gas stream to a gas chromatographic analytical unit.

The product gas stream leaving the back pressure regulator (15) was measured and samples were withdrawn and analysed by gas chromatography. The liquid product was also sampled and analysed by gas chromatography.

When the reactor had reached equilibrium a balanced run was carried out over a one hour period at a temperature of 400°C. The results obtained are given in Table 4.

TABLE 2
X-ray diffraction data for modified crystalline silica product of Example 1

| 2-Theta | D(A°) | I/Io |
|---------|--------|------|
| 7.41 | 11.935 | 5 |
| 8.07 | 10.960 | 72 |
| 8.64 | 10.236 | 20 |
| 8.95 | 9.884 | 100 |
| 9.02 | 9.799 | 98 |
| 9.23 | 9.584 | 14 |
| 14.09 | 6.289 | 6 |
| 14.93 | 5.932 | 14 |
| 15.11 | 5.864 | 7 |
| 15.71 | 5.640 | 7 |
| 16.05 | 5.521 | 6 |
| 17.80 | 4.983 | 5 |
| 18.00 | 4.929 | 8 |
| 21.03 | 4.224 | 7 |
| 22.32 | 3.983 | 4 |
| 23.22 | 3.830 | 54 |
| 23.44 | 3.795 | 33 |
| 23.49 | 3.786 | 33 |
| 23.87 | 3.728 | 17 |
| 24.10 | 3.693 | 17 |
| 24.49 | 3.635 | 6 |
| 24.70 | 3.604 | 6 |
| 26.10 | 3.415 | 4 |
| 26.76 | 3.332 | 4 |
| 27.05 | 3.296 | 6 |
| 29.54 | 3.024 | 4 |
| 30.04 | 2.975 | 7 |
| 30.35 | 2.945 | 4 |

TABLE 3
X-ray diffraction data for modified crystalline silica product of Example 2

| 2-Theta | D(A°) | I/Io |
|---------|--------|------|
| 7.26 | 12.180 | 4 |
| 7.55 | 11.715 | 9 |
| 7.76 | 11.398 | 30 |
| 8.03 | 11.012 | 100 |
| 8.36 | 10.574 | 6 |
| 8.89 | 9.950 | 77 |
| 8.98 | 9.845 | 63 |
| 9.18 | 9.632 | 16 |
| 13.32 | 6.648 | 7 |
| 14.05 | 6.305 | 10 |
| 14.90 | 5.947 | 14 |
| 15.07 | 5.878 | 4 |
| 15.61 | 5.677 | 6 |
| 15.71 | 5.640 | 6 |
| 16.01 | 5.535 | 7 |
| 17.77 | 4.993 | 5 |
| 17.95 | 4.940 | 6 |
| 20.49 | 4.334 | 5 |
| 21.00 | 4.231 | 6 |
| 23.18 | 3.837 | 55 |
| 23.38 | 3.806 | 26 |
| 23.47 | 3.790 | 25 |
| 23.84 | 3.732 | 16 |
| 24.07 | 3.698 | 23 |
| 24.44 | 3.643 | 8 |
| 24.69 | 3.605 | 8 |
| 25.96 | 3.433 | 4 |
| 26.09 | 3.416 | 4 |

**0 050 525**

TABLE 3 (contd.)
X-ray diffraction data for modified crystalline silica product of Example 2

| 2-Theta | D(A°) | I/Io |
|---------|-------|------|
| 26.73 | 3.336 | 4 |
| 27.01 | 3.301 | 5 |
| 27.13 | 3.287 | 6 |
| 30.03 | 2.976 | 10 |
| 30.34 | 2.946 | 5 |

TABLE 4
Reaction parameters:
GHSV=4650 hr$^{-1}$
$H_2$:CO molar ratio=2:1
Pressure=50 bar
Recycle ratio=nil

| Reaction temperature °C | CO conversion % | Carbon dioxide | Selectivity % | | | |
|---|---|---|---|---|---|---|
| | | | Methane | Ethane | Propane | Methanol |
| 400 | 5 | 29 | 44 | 10 | 5 | 12 |

## Claims

1. A synthetic modified crystalline silica comprising crystalline silica in which a proportion of the silicon atoms in the crystal lattice have been replaced by nickel, which modified crystalline silica has an X-ray diffraction pattern characterised by the lines as set forth below

| D (Angstrom) | I/Io |
|---|---|
| 11.0 ±0.2 | S |
| 10.0 ±0.2 | S |
| 9.8 ±0.2 | S |
| 9.2 ±0.2 | W |
| 6.3 ±0.1 | VW |
| 5.9 ±0.1 | W |
| 5.85±0.1 | VW |
| 5.64±0.1 | VW |
| 5.50±0.1 | VW |
| 5.00±0.08 | VW |
| 4.90±0.08 | VW |
| 4.22±0.08 | VW |
| 3.83±0.07 | M |
| 3.80±0.07 | M |
| 3.79±0.07 | M |
| 3.73±0.05 | W |
| 3.69±0.05 | W |
| 3.64±0.05 | VW |
| 3.60±0.05 | VW |
| 3.42±0.05 | VW |
| 3.33±0.04 | VW |
| 3.30±0.04 | VW |
| 2.98±0.02 | VW |
| 2.95±0.02 | VW |

2. A process for the production of the synthetic modified crystalline silica as claimed in claim 1 which process comprises mixing, in a liquid medium comprising either water, an alcohol, or a mixture thereof, a source of silica, a source of nickel and an organic base in the absence of an alkali or alkaline earth metal compound, maintaining the mixture at elevated temperature for a time sufficient to effect crystallisation of the modified crystalline silica and recovering the modified crystalline silica so-formed.

3. A process according to claim 2, wherein the organic base is a tetraalkylammonium compound in which the alkyl group contains from 1 to 5 carbon atoms.

4. A process according to either claim 2 or claim 3, wherein the recovered modified crystalline silica is washed with boiling distilled water having dissolved therein an ammonium salt.

7

5. A process according to any one of claims 2 to 4, wherein the recovered modified crystalline silica is calcined by heating in air at a temperature in the range from 300 to 700°C for a period of from 2 to 24 hours.

6. A process for the production of synthesis gas from methanol which comprises contacting at elevated temperature methanol in the vapour phase with a catalyst comprising the nickel-modified crystalline silica as claimed in claim 1.

7. A process according to claim 6, wherein there is added to the nickel-modified crystalline silica a metal of Group VIII of the Periodic Table either alone or in combination with one or more other metals of Groups I to VIII of the Periodic Table.

8. A process according to claim 7, wherein the Group VIII metal is rhodium and/or cobalt.

9. A process according to claim 7, wherein the Group VIII metal is admixed with one or more of the metals iron, copper, chromium, gold and zinc.

10. A process according to any one of claims 7 to 9, wherein there is added to the nickel-modified crystalline silica a mixture of rhodium and copper.

11. A process according to any one of claims 6 to 10, wherein the temperature is in the range from 300 to 450°C and the pressure is in the range from 1 to 50 atmospheres.

12. A process for the conversion of synthesis gas to hydrocarbons and/or oxygenated hydrocarbons which process comprises contacting synthesis gas at elevated temperature and pressure with a catalyst comprising the nickel-modified crystalline silica as claimed in claim 1.

13. A process according to claim 12, wherein the temperature is in the range from 200 to 400°C and the pressure is in the range from 10 to 300 bars.

14. A process according to either claim 12 or claim 13, wherein there is added to the modified crystalline silica one or more of the metals cobalt, iron, manganese, nickel, ruthenium, rhodium, palladium, zinc, chromium and copper.

## Patentansprüche

1. Synthetisch modifizierte kristalline Kieselsäure, enthaltend kristalline Kieselsäure, in welcher ein Teil der Siliciumatome im kristallinen Gitter durch Nickel ersetzt ist, wobei die modifizierte kristallinen Kieselsäure ein Röntgen-Brechungsmuster hat, das durch die folgenden Linien charakterisiert ist:

| D (Angström) | I/Io |
|---|---|
| 11,0 ±0,2 | S |
| 10,0 ±0,2 | S |
| 9,8 ±0,2 | S |
| 9,2 ±0,2 | W |
| 6,3 ±0,1 | VW |
| 5,9 ±0,1 | W |
| 5,85±0,1 | VW |
| 5,64±0,1 | VW |
| 5,50±0,1 | VW |
| 5,00±0,08 | VW |
| 4,90±0,08 | VW |
| 4,22±0,08 | VW |
| 3,83±0,07 | M |
| 3,80±0,07 | M |
| 3,79±0,07 | M |
| 3,73±0,05 | W |
| 3,69±0,05 | W |
| 3,64±0,05 | VW |
| 3,60±0,05 | VW |
| 3,42±0,05 | VW |
| 3,33±0,04 | VW |
| 3,30±0,04 | VW |
| 2,98±0,02 | VW |
| 2,95±0,02 | VW |

(wobei S=stark, M=mittel, W=schwach und VW=sehr schwach bedeutet).

2. Verfahren zur Herstellung der synthetisch modifizierten kristallinen Kieselsäure gemäß Anspruch 1, dadurch gekennzeichnet, daß man in einem flüssigen, Wasser, einen Alkohol oder eine Mischung derselben enthaltenden Medium eine Kieselsäurequelle, eine Nickelquelle und eine organische Base in Abwesenheit einer Alkali- oder Erdalkalimetallverbindung mischt, die Mischung auf eine zum Bewirken der Kristallisation der modifizierten kristallinen Kieselsäure ausreichende Zeit auf erhöhter Temperatur hält und die so gebildete modifizierte kristalline Kieselsäure gewinnt.

# 0 050 525

3. Verfahren gemäß Anspruch 2, in welchem die organische Base eine Tetralkylammoniumverbindung ist, in welcher die Alkylgruppe 1 bis 5 Kohlenstoffatome enthält.

4. Verfahren gemäß Anspruch 2 oder 3, in welchem die gewonnene modifizierte kristalline Kieselsäure mit siedendem destilliertem Wasser gewaschen wird, in welchem ein Ammoniumsalz gelöst ist.

5. Verfahren gemäß einem der Ansprüche 2 bis 4, in welchem die gewonnene modifizierte kristalline Kieselsäure durch Erhitzen in Luft bei einer Temperatur im Bereich von 300 bis 700°C für eine Dauer von 2 bis 24 Stunden calciniert wird.

6. Verfahren zur Herstellung von Synthesegas aus Methanol, dadurch gekennzeichnet, daß man bei erhöhter Temperatur Methanol in der Dampfphase mit einem Katalysator im Berührung bringt, der die nickelmodifizierte kristalline Kieselsäure gemäß Anspruch 1 enthält.

7. Verfahren gemäß Anspruch 6, in welchem man zur nickelmodifizierten kristallinen Kieselsäure ein Metall der Gruppe VIII des Periodischen Systems allein oder in Kombination mit einem oder mehreren anderen Metallen der Gruppen I bis VIII des Periodischen Systems zufügt.

8. Verfahren gemäß Anspruch 7, in welchem das Metall der Gruppe VIII Rhodium und/oder Kobalt ist.

9. Verfahren gemäß Anspruch 7, in welchem das Metall der Gruppe VIII mit einem oder mehreren der Metalle Eisen, Kupfer, Chrom, Gold und Zink gemischt wird.

10. Verfahren gemäß einem der Ansprüche 7 bis 9, in welchem zur nickelmodifizierten kristallinen Kieselsäure eine Mischung aus Rhodium und Kupfer zugefügt wird.

11. Verfahren gemäß einem der Ansprüche 6 bis 10, in welchem die Temperatur im Bereich von 300 bis 450°C und der Druck im Bereich von 1 bis 50 at liegt.

12. Verfahren zur Umwandlung von Synthesegas in Kohlwasserstoffe und/oder oxygenierte Kohlenwasserstoffe, dadurch gekennzeichnet, daß man Synthesegas bei erhöhter Temperatur und Druck mit einem Katalysator in Berührung bringt, der die nickelmodifizierte kristalline Kieselsäure gemäß Anspruch 1 enthält.

13. Verfahren gemäß Anspruch 12, in welchem die Temperatur im Bereich von 200 bis 400°C und der Druck im Bereich von 10 bis 300 bar liegt.

14. Verfahren gemäß Anspruch 12 oder 13, in welchem zu der modifizierten kristallinen Kieselsäure ein oder mehrere der Metalle Kobalt, Eisen, Mangan, Nickel, Ruthenium, Rhodium, Palladium, Zink, Chrom und Kupfer zugegeben wird bzw. werden.

**Revendications**

1. Silice cristalline synthétique modifiée, comprenant de la silice cristalline dans laquelle une certaine proportion des atomes de silicium du réseau cristallin ont été remplacés par du nickel, cette silice cristalline modifiée ayant un spectre de diffraction des rayons X caractérisé par les raies indiquées ci après:

| D (Angstrom) | $I/I_o$ |
|---|---|
| 11,0 ±0,2 | F |
| 10,0 ±0,2 | F |
| 9,8 ±0,2 | F |
| 9,2 ±0,2 | f |
| 6,3 ±0,1 | tf |
| 5,9 ±0,1 | f |
| 5,85±0,1 | tf |
| 5,64±0,1 | tf |
| 5,50±0,1 | tf |
| 5,00±0,08 | tf |
| 4,90±0,08 | tf |
| 4,22±0,08 | tf |
| 3,83±0,07 | M |
| 3,80±0,07 | M |
| 3,79±0,07 | M |
| 3,73±0,05 | f |
| 3,69±0,05 | f |
| 3,64±0,05 | tf |
| 3,60±0,05 | tf |
| 3,42±0,05 | tf |
| 3,33±0,04 | tf |
| 3,30±0,04 | tf |
| 2,98±0,02 | tf |
| 2,95±0,02 | tf |

# 0 050 525

2. Procédé de production de la silice cristalline synthétique modifiée selon la revendication 1, ce procédé comprenant le mélange, dans un milieu liquide comprenant de l'eau, un alcool ou un de leurs mélanges, d'une source de silice, d'une source de nickel et d'une base organique en l'absence d'un composé de métal alcalin ou alcalino terreux, le maintien du mélange à température élevée pendant un temps suffisant pour réaliser la cristallisation de la silice cristalline modifiée, et la récupération de la silice cristalline modifiée ainsi formée.

3. Procédé selon la revendication 2, dans lequel la base organique est un composé de tétraalkyl-ammonium dans lequel le groupe alkyle contient de 1 à 5 atomes de carbone.

4. Procédé selon la revendication 2 ou la revendication 3, dans lequel la silice cristalline modifiée récupérée est lavée à l'aide d'eau distillée bouillante comportant en dissolution un sel d'ammonium.

5. Procédé selon l'une quelconque des revendications 2 à 4, dans lequel la silice cristalline modifiée récupérée est calcinée par chauffage à l'air à une température comprise entre 300 et 700°C pendant une période de 2 à 24 heures.

6. Procédé pour la production de gaz de synthèse à partir de méthanol, qui comprend la mise en contact, à température élevée, du méthanol en phase vapeur avec un catalyseur comprenant la silice cristalline modifiée par du nickel selon la revendication 1.

7. Procédé selon la revendication 6, dans lequel il est ajouté à la silice cristalline modifiée par du nickel un métal du groupe VIII du Tableau Périodique, seul ou en combinaison avec un ou plusieurs autres métaux des groupes I à VIII du Tableau Périodique.

8. Procédé selon la revendication 7, dans lequel le métal du groupe VIII est du rhodium et/ou du cobalt.

9. Procédé selon la revendication 7, dans lequel le métal du groupe VIII est mélangé à un ou plusieurs des métaux fer, cuivre, chrome, or et zinc.

10. Procédé selon l'une quelconque des revendications 7 à 9, dans lequel on ajoute à la silice cristalline modifiée par du nickel un mélange de rhodium et de cuivre.

11. Procédé selon l'une quelconque des revendications 6 à 10, dans lequel la température se situe entre 300 et 450°C et la pression se situe entre 1 et 50 atmosphères.

12. Procédé pour la transformation du gaz de synthèse en des hydrocarbures et/ou des hydrocarbures oxygénés, ce procédé comprenant la mise en contact de gaz de synthèse, à température et pression élevées, avec un catalyseur constitué par, ou comprenant, la silice cristalline modifiée par du nickel selon la revendication 1.

13. Procédé selon la revendication 12, dans lequel la température se situe entre 200 et 400°C et la pression entre 10 et 300 bars.

14. Procédé selon la revendication 12 ou la revendication 13, dans lequel on ajoute, à la silice cristalline modifiée, un ou plusieurs des métaux cobalt, fer, manganèse, nickel, ruthénium, rhodium, palladium, zinc, chrome et cuivre.

10

INLET MANIFOLD

WATER WASH

PRODUCT GAS

WAX PRODUCT

AQUEOUS PRODUCT

RECYCLE GAS

1
2
3
4
5
6
7
8
9
10
11
12
13
14
15
16
17
18
19